# EUROPEAN PATENT SPECIFICATION

(11) **EP 1 665 984 B1**
(45) Date of publication and mention of the grant of the patent: **17.10.2007**
(21) Application number: 06075602.0
(22) Date of filing: 02.05.1996
(51) Int. Cl.: A61B 5/11, A01K 11/00

(54) **A method of automatically milking animals and monitoring their state of health**
Verfahren zum automatischen Melken von Tieren und zur Überwachung ihres Gesundheitszustandes
Méthode de traite automatique d'animaux et de surveillance de leur état de santé

(30) Priority: 17.05.1995 NL 1000380
(43) Date of publication of application: 07.06.2006
(62) Divisional of application: 03076259.5
(73) Proprietor: MAASLAND N.V., 3147 PA Maassluis (NL)
(72) Inventor: van der Lely, Alexander, 3065 NA Rotterdam (NL); van den Berg, Karel, 2971 BR Bleskensgraaf (NL); van der Lely, Olaf, 6300 Zug (CH)
(74) Representative: Corten, Maurice Jean F.M.

(56) References cited:
- EP-A- 0 638 231
- WO-A-94/19931
- US-A- 4 247 758
- US-A- 4 618 861

## Description

The invention relates to a method of automatically milking animals that are allowed to walk freely in an accommodation, such as a stable or cowshed or a meadow, and to go individually to a milking parlour including a milking robot where, after having been identified, they may be milked.

Such a method is known from US-A-4 247 758.

It is an object of the invention to provide an alternative method.

The present invention provides a method according to claim 1. Preferably, there is thereby determined in the computer, on the basis of the activity pattern established for individual animals, an average value over a fixed period of time, and the most recently detected activity pattern is compared with said average value, while, with the aid of this comparison, there is ascertained whether an animal is on heat, is ill or has contracted a latent disease, or is injured. As such an average value will differ depending on the fact whether the animal is in a stable or cowshed or in a meadow, the method is furthermore characterized in that this average value is recorded in the computer both for the periods of time during which the animal stays in a stable or cowshed and for the periods of time during which the animal stays in a meadow.

The activity pattern is established by means of counting the movements of an animal. In particular, when an animal presents itself in the milking parlour or near thereto, said animal will only be milked if the sum of the counts exceeds a fixed value.

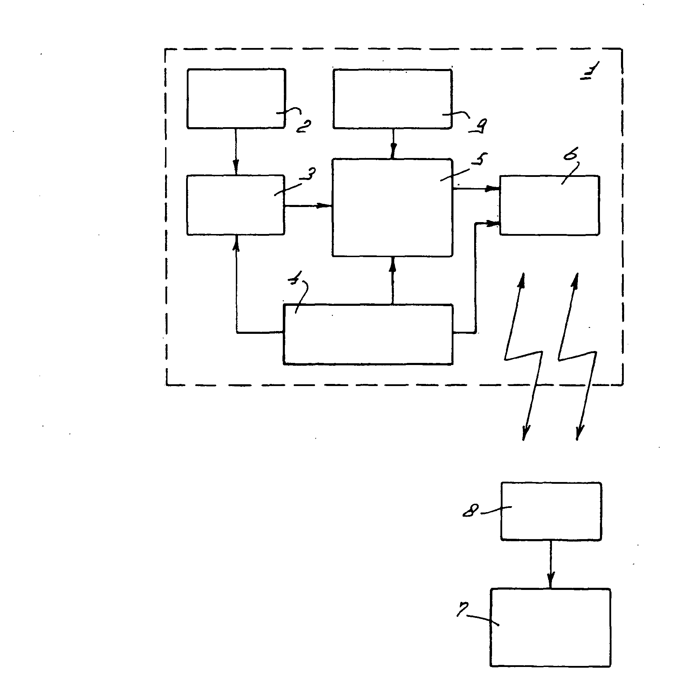

For a better understanding of the invention and to show how the same may be carried into effect, reference will now be made, by way of example, to the accompanying drawing, in which the animal activity meter according to the invention is shown schematically.

The animal activity meter 1 shown in the drawing, which meter can be worn by an animal around the neck or attached to one of the legs, is provided with a movement sensor 2, for the purpose of detecting the movements made by the animal. Said movement sensor 2 may be designed as a mercury switch. The number of movements established by means of the movement sensor 2 can be counted in a counting device 3, which is controlled by a control circuit 4 provided with a microprocessor. This control circuit ensures that the counting device 3 is reset in its starting position and will count thereafter, during a pre-fixed period of time, the number of movements made in said period of time which, optionally, can be adjusted, e.g. at five minutes, a quarter of an hour, half an hour, or at whatever value. After the adjusted period of time has elapsed, the count of the counting device is recorded in memory means 5, which are also controlled by the control circuit 4. Every time a count from the counting device 3 has been recorded in the memory means 5, the counting device 3 is reset in its starting position by the control circuit 4. In this manner, after some time, there is stored a series of counts in the memory means 5. Moreover, the animal activity meter 1 includes a transponder 6, by means of which the counts stored in the memory means 5 can be recorded in a computer 7, via a sensor 8 connected thereto. Although the sensor 8 may be arranged at a place chosen at random in a stable or cowshed, said sensor should preferably be disposed at the place where the animals are milked or near thereto, as this is the place where the animals go or are guided a number of times per twenty-four hours. As soon as the animals come in the vicinity of the sensor 8, the latter comes in contact with the transponder 6 in the animal activity meter 1, while the information present in the memory means 5 can be transmitted and recorded in the computer 7 via the sensor 8. For the purpose of supplying concentrate or for automatically milking, it is desirable that the animals can be identified at the place where they are fed or milked or near thereto. This identification is effected by means of an animal identification system provided with a transponder 6 including an electronic circuit, to be worn by the animal, in which system the animal number has been recorded, and a fixed sensor being in communication with a computer. It will be obvious that the animal activity meter and the animal identification system are adapted to be mutually integrated. For that purpose, in the embodiment shown, the memory means 5, besides the counts, also contain the animal number and, as soon as the animal comes in the vicinity of the sensor 8, these two data, via the transponder 6 and said sensor 8, are recorded in the computer 7. For each animal, there may be in the memory of the computer 7 a file, that is accessible with the aid of said animal number and in which the counts are stored. This file may additionally include all kinds of information relevant for feeding the animals or for automatically milking same.

When the animals are automatically milked while making use of a milking robot, the animals allowed to walk freely in an accommodation, such as a stable or cowshed or a meadow, can go individually, without human intervention, to the milking parlour in which the milking robot is disposed, where, after having been identified by means of the animal identification system, they may be milked. As there is usually supplied concentrate to the animals in the milking parlour, and the animals can go there voluntarily, it will be obvious that not every time the animals present themselves in the milking parlour they will actually be milked. It is possible to let the decision whether or not to milk an animal depend on the activity pattern as is established by means of the animal activity meter 1, as, for each animal, the activity pattern between two consecutive moments when the animal presents itself in the milking parlour or near thereto is established and recorded in the computer 7. When, every time the animal has presented itself in the milking parlour or near thereto, a series of counts is read out, there is obtained a permanently updated activity pattern. After having been milked, an animal will be in movement and consume fodder. After some time, the animal will lie down and ruminate. When, thereafter, the animal rises and begins to move, its movements will be less frequent than in the period prior to ruminating, as the lactation has sufficiently been resumed and the udder has become heavy, so that the freedom of movement of the animal is hampered. When the animal then goes again to the milking parlour, it will be milked. However, when the animal goes to the milking parlour and it appears from the activity pattern that this animal has not yet ruminated, then said animal has obviously presented itself too early and has to be removed from the milking parlour without having been milked. In other words, on the basis of the updated activity pattern, there can be decided whether or not to milk the animal when the latter presents itself in the milking parlour or near thereto. During the period of time the animal is lying down and is possibly ruminating, its movements are minimal. During that time, it is not strictly necessary that the counting device 3 is active and that the count is periodically recorded from said counting device in the memory means 5. The animal activity meter 1 may be provided with a specific sensor 9 for the purpose of measuring the distance to the ground and inciting a signal indicating that the animal is lying, standing or walking. The signals incited by this distance measuring sensor determine the moments when the animal lies down and rises, and can be recorded in the memory means 5. In other words, the periods of rest of the animal, obtained by means of the distance measuring sensor 9, can be stored directly in the memory means 5. These data can be recorded, in the same manner as the counts and the animal number, from the memory means 5, via the transponder 6 and the sensor 8, in the computer 7.

Besides directly from the updated activity pattern, the decision whether or not to milk an animal presenting itself in the milking parlour or near thereto can also be taken depending on the fact whether or not the sum of the counts, recorded in the computer 7 from the moment when the animal has been milked the previous time, exceeds a fixed value. After all, in the computer 7 there can be recorded how many movements a specific animal normally makes from the moment it has been milked until it has subsequently consumed fodder and has ruminated same, so that, as soon as an animal presents itself in the milking parlour or near thereto, the sum of the counts obtained since the previous milking run can be ascertained and the decision to milk the animal can be taken when this sum exceeds the threshold value for the number of movements to be made for this animal recorded in the computer.

The activity pattern detected for the individual animals can be averaged over a number of days. For example, by constantly making an average over the last week, there is obtained a possibly slowly varying value of a developing average. The animal activity as is read out from the memory means 5 every time the animal presents itself in the milking parlour or near thereto, can be compared with this developing average. In the case of values obtained that are significantly higher, the animal will be supposed to be on heat and in the case of values obtained being significantly lower, there will be an indication that the animal has one or more injured legs and/or is ill or at least has contracted a disease. Upon comparing the animal activity with the average value recorded in the memory, it has to be taken into consideration that these average values will not only differ per animal, but also depend on the fact whether the animal stays in a stable or cowshed or in a meadow. This means that for each animal there can be updated an average value for the period of time during which it stays in the stable or cowshed, as well as for the period of time during which it stays in the meadow.

## Claims

1. A method of automatically milking and monitoring the state of health of animals that are allowed to walk freely in an accommodation, such as a stable or cowshed or a meadow, and to go individually to a milking parlour including a milking robot where, after having been identified, they may be milked, **characterized in that**, for individual animals, the activity pattern between two moments when an animal presents itself in the milking parlour or near thereto is established by means of an activity meter (1) based on counting the movements of the animal, and recorded in a computer (7), whereafter, on the basis of this activity pattern, there is indicated by the computer the state of health of the animal, in particular whether the animal is on heat, is ill or has contracted a latent disease, or is injured, and **in that**, when an animal presents itself in the milking parlour or near thereto, said animal will only be milked when the sum of the counts from the moment when the animal has been milked the previous time exceeds a fixed value.

2. A method as claimed in claim 1, **characterized in that** a series of counts is recorded as the activity pattern, wherein a count is the number of movements during a pro-fixed period of time.

3. A method as claimed in claim 1 or 2, **characterized in that** in the computer, on the basis of the activity pattern established for individual animals, an average value over a fixed period of time is determined and the most recently detected activity pattern is compared with said average value, while, with the aid of this comparison, there is ascertained whether an animal is on heat, is ill or has contracted a latent disease, or is injured.

4. A method as claimed in claim 3, **characterized in that** this average value is recorded in the computer both for the periods of time during which the animal stays in a stable or cowshed and for the periods of time during which the animal stays in a meadow.

## Patentansprüche

1. Verfahren zum automatischen Melken und Überwachen des Gesundheitszustandes von Tieren, die in einer Unterkunft, wie z. B. einem Stall oder Kuhstall, oder auf einer Wiese frei umherlaufen und einzeln zu einem Melkstand mit einem Melkroboter gehen können, wo sie, nachdem sie identifiziert worden sind, gemolken werden können,
**dadurch gekennzeichnet, dass** für einzelne Tiere das Aktivitätsmuster zwischen zwei Zeitpunkten, zu denen sich ein Tier bei oder nahe dem Melkstand einfindet, mit Hilfe eines Aktivitätsmessers (1), der auf dem Zählen der Bewegungen des Tieres basiert, erstellt und in einem Computer (7) aufgezeichnet wird, wonach auf der Basis dieses Aktivitätsmusters von dem Computer der Gesundheitszustand des Tieres angezeigt wird, insbesondere, ob das Tier brünstig ist, krank ist oder eine latente Krankheit hat oder verletzt ist, und dass sich beim Einfinden eines Tieres bei oder nahe dem Melkstand das Tier nur dann gemolken wird, wenn die Summe der Zählwerte seit dem Zeitpunkt des letzten Melkens des Tieres einen festgelegten Wert überschreitet.

2. Verfahren nach Anspruch 1,
**dadurch gekennzeichnet, dass** als Aktivitätsmuster eine Anzahl von Zählwerten aufgezeichnet wird, wobei ein Zählwert die Anzahl von Bewegungen während eines vorgegebenen Zeitraumes ist.

3. Verfahren nach Anspruch 1 oder 2,
**dadurch gekennzeichnet, dass** in dem Computer auf der Basis des für einzelne Tiere ermittelten Aktivitätsmusters ein Durchschnittswert über einen festgelegten Zeitraum ermittelt wird und das zuletzt ermittelte Aktivitätsmuster mit dem Durchschnittswert verglichen wird, wobei mittels dieses Vergleiches festgestellt wird, ob ein Tier brünstig ist, krank ist oder eine latente Krankheit hat oder verletzt ist.

4. Verfahren nach Anspruch 3,
**dadurch gekennzeichnet, dass** dieser Durchschnittswert sowohl für die Zeiträume, in denen sich das Tier in einem Stall oder Kuhstall aufhält, als auch für die Zeiträume, in denen sich das Tier auf einer Wiese aufhält, in dem Computer gespeichert wird.

## Revendications

1. Procédé de traite automatique et de surveillance de l'état de santé des animaux qui sont autorisés à se déplacer librement dans un logement, tel qu'une étable ou un pré, et à se rendre individuellement à une salle de traite comportant un robot de traite où, après avoir été identifiés, ils peuvent être traits, **caractérisé en ce que**, pour des animaux individuels, le modèle d'activité entre deux moments lorsqu'un animal se présente à la salle de traite ou à proximité de celle-ci est établi au moyen d'un dispositif de mesure d'activité (1) en se basant sur le comptage des mouvements de l'animal, et enregistré dans un ordinateur (7), après quoi, sur la base de ce modèle d'activité, l'ordinateur indique l'état de santé de l'animal, en particulier si l'animal est en chaleur, est malade ou a contracté une maladie latente, ou est blessé, et **en ce que**, lorsqu'un animal se présente à la salle de traite ou à proximité de celle-ci, ledit animal ne sera trait que lorsque la somme des décomptes à partir du moment où l'animal a été trait la fois précédente dépasse une valeur fixée.

2. Procédé selon la revendication 1, **caractérisé en ce qu'**une série de décomptes est enregistrée comme modèle d'activité, dans lequel un décompte est le nombre de mouvements pendant une période de temps préfixée.

3. Procédé selon la revendication 1 ou 2, **caractérisé en ce que** dans l'ordinateur, sur la base du modèle d'activité établi pour des animaux individuels, une valeur moyenne pendant une période de temps fixée est déterminée et le modèle d'activité le plus récemment détecté est comparé à ladite valeur moyenne, tandis que, à l'aide de cette comparaison, on détermine si un animal est en chaleur, est malade ou a contracté une maladie latente, ou est blessé.

4. Procédé selon la revendication 3, **caractérisé en ce que** cette valeur moyenne est enregistrée dans l'ordinateur à la fois pour les périodes de temps pendant lesquelles l'animal reste dans une étable ou une vacherie ou pour les périodes durant lesquelles l'animal reste dans un pré.
